# EUROPEAN PATENT APPLICATION

(11) **EP 4 302 694 A1**
(43) Date of publication of application: **10.01.2024**
(21) Application number: 22183787.5
(22) Date of filing: 08.07.2022
(51) Int. Cl.: A61B 6/04, A61B 6/00, A61B 8/00

(54) **SYSTEM AND METHOD FOR PARALLEL PREPARATION OF MULTIPLE PATIENTS**

(71) Applicant: Koninklijke Philips N.V., 5656 AG Eindhoven (NL)
(72) Inventor: LEUSSLER, Christoph Günther, Eindhoven (NL); WUELBERN, Jan Hendrik, Eindhoven (NL); BUSSA, Nagaraju, 5656AG Eindhoven (NL); WEISS, Steffen, 5656AG Eindhoven (NL); PRABHU, Rajesh, Eindhoven (NL)
(74) Representative: Philips Intellectual Property & Standards

(57) **Abstract**

The present invention relates to a patient bed for accommodating a patient in a medical imaging system. The patient bed comprises a table top configured for receiving the patient and an interface module comprising a first interface and a second interface. The first interface is configured to be communicationally connected to at least one medical device attached to the patient, and the second interface is configured to be communicationally connected to a control unit for communicating with the at least one medical device. Thus, it is possible to prepare multiple patients in parallel on multiple patient beds before transporting the patients to the medical imaging apparatus. Sensors and coils can be attached to the patient and there functionality can be tested in advance before e4ntering the scanner room for medical imaging.

## Description

### FIELD OF THE INVENTION

The present invention relates to a patient bed for accommodating a patient in a medical imaging system. The patient bed is configured to allow parallel preparation of multiple patients for medical imaging.

### BACKGROUND OF THE INVENTION

Preparation of multiple patients in parallel and guided imaging is a challenge regarding workflow management and technical realization in a clinical environment. Parallel preparation of patients allows to scan the patients with reduced latency as the patients are actively prepared before entering the scanner room and thus are ready to scan. With respect to the workflow aspects for imaging of multiple patient with one scanner, there exist many problems. A long preparation time of the patient at the scanner and sequential preparation of the patients may lead to latency and lower patient throughput through the medical imaging system. When docking of the patient table to the imaging system, coils and sensors need to be manually connected to the imaging system, which further increases the latency. For multiple patient tables and a table docking system, the integration of coils and physiological sensors for the clinical application or scan protocol needs to be realized.

The inventors of the present invention have thus found that it would be advantageous to have a patient bed that allows parallel preparation of multiple patients that does not suffer from the above-mentioned drawbacks.

### SUMMARY OF THE INVENTION

It is an object of the present invention to provide an improved patient bed for accommodating a patient in a medical imaging system that allows for parallel preparation of multiple patients before entering the scanner room.

The object of the present invention is solved by the subject matter of the independent claims, wherein further embodiments are incorporated in the dependent claims.

The described embodiments similarly pertain to the patient bed for accommodating a patient in a medical imaging system and the method of imaging a patient in a medical imaging system with the patient bed. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first embodiment of the invention, there is provided a patient bed for accommodating a patient in a medical imaging system. The patient bed comprises a table top configured for receiving the patient and an interface module comprising a first interface and a second interface. The first interface is configured to be communicationally connected to at least one medical device attached to the patient, and the second interface is configured to be communicationally connected to a control unit for communicating with the at least one medical device.

With an intelligent modular patient bed according to the invention, the preparation of multiple patients in parallel on several such patient beds with coils and sensors is enabled. The device functionality and physiological signal response of each of the coils or sensors can be already checked and the signals of each patient can be transmitted before the patient prepared for scanning enters the scanner room.

The proposed patient bed is equipped with connectors allowing galvanic, optical or wireless connection of devices to the patient bed. In addition, the patient bed allows smart galvanic, optical or wireless connection to a control unit of an imaging system or a trolley-Preferably, the connection to the control unit is established without active interaction of an operator like clinic personnel. For the autonomous connection and setup, the patient bed can automatically connect to the control unit or diagnostic scanner controlled by 3D sensing and a software module. The software module can be based on artificial intelligence and optimizes and guides the parallel patient preparation and allocation to a diagnostic or therapy device.

Preferably, a software algorithm controls the workflow of parallel patient preparation and imaging. Different software modules can be active in parallel and interact with each other, and control and exchange data. Transportation, docking, and scanning rely on parameters such as control of software update, calibration, data connection quality, inter data flow, or serviceability of connected devices. These parameters can be real time checked by a threshold or window based decision management. Preferably, a software management system can make an active link between the sensors and coils, the patient and the imaging system. For parallel and partly autonomous imaging support there may be a larger selection option in order to better match the patient profile or the coil profile. This process can be autonomous, and in addition or alternatively, there may be a backup with staff support. In addition, a patient queue for multiple scanners can be managed. The patient bed according to the invention can comprise a clear smart active identification sensing for multiple patient preparation.

Thus, multiple patients can be prepared in parallel for medical imaging on multiple patient beds according to the invention. Each patient can be arranged for medical imaging on a respective table top of the patient bed. The patient can be provided with several medical devices like sensors detecting physiological signals of the patient, with equipment related to the imaging procedure like coils of a magnetic resonance imaging system, or devices for treatment of the patient. This medical devices provided to the patient can be connected to the patient bed via the first interface of the interface module of the patient bed. The second interface of the interface module of the patient bed allows connection of the patient bed to an external control unit. The control unit allows to check and test the functionality of the medical devices like sensors and coils in advance before the patient bed with the patient is transferred to the medical imaging system. Therefore, the patient bed according to the invention reduces latency and idle time of the medical imaging system thus increasing patient throughput and reducing costs.

Thus, the patient bed according to the invention proposes a modular patient table design, which helps the operator and patients in the pipeline to get prepared in advance before the actual scanning. A smart table top with connections to the individual coils and sensors enables the test of the medical devices prior to their use in the medical imaging system. Therefore, cost can be reduced and staff and patient satisfaction can be improved.

In an embodiment of the invention, the control unit is part of the medical imaging system, or the control unit is part of a trolley for transporting the patient bed. Thus, the control unit can be part of a trolley configured for transporting the patient bed, or part of the medical imaging system. Alternatively, there can also be a separate control unit, or a control unit integrated into the patient bed. The control unit allows readout of the medical devices and therefore a test of the sensor signals and the functionality of the coils. A manual connection of the electrical coils or sensors directly to the imaging system can be avoided.

In an embodiment of the invention, the second interface is configured to disconnect from the trolley and to connect with the medical imaging system when the patient bed is moved from the trolley to the medical imaging system. The interface module with the second interface can be configured such that the connection of the second interface is automatically changed from the trolley to the imaging module. After successfully checking the device functionality and transporting the patient to the imaging system, the connection to the trolley is no longer necessary. Thus, it is disconnected, and a new connection of the second interface to the medical imaging system is established. Thus, the medical imaging can control, preferably via the control unit, the coils and other medical devices like sensors measuring physiological signals of the patient.

In an embodiment of the invention, the at least one medical device is a physiological sensor for detecting a body function of the patient, a coil of the medical imaging system, or a device for treating the patient. The medical devices can also be devices and gadgets such as RF coils, sensors for ECG, PPU, or respiration, contrast injectors, or injectors for sedation, oxygen supply of patient, blood vessel access for sedation and stabilization, etc.

In an embodiment of the invention, the second interface is configured to establish the connection to the control unit automatically. In this embodiment, there is preferably no active input and no manual work from an operator necessary to establish the connection. Thus, when the patient bed is transferred from the trolley to the imaging system, or when the patient bed is inserted into the imaging system, the connection is established automatically.

In an embodiment of the invention, the connection to the control unit is established using 3D sensing. This three-dimensional sensing can be used to detect the position and/or orientation of the second interface of the patient bed with respect to the medical imaging system. Thus, a connector of the control unit of the medical imaging system can be controlled to adapt its position to connect with the second interface of the interface module, thus establishing a connection between the medical devices attached to the patient and the medical imaging system. In an embodiment of the invention, the connection of the first interface with the at least one medical device and/or the connection of the second interface with the control unit is a galvanic, an optical, or a wireless connection. The connection can be galvanic, thus referring to a direct electrical contact of cables via a connector. Alternatively, an optical connection like a light guide can be provided, which can be able to transfer the control and data signals via small gap between the interface module and the imaging system. A wireless connection can use RF transmission for contactless transfer of the signals.

In an embodiment of the invention, the interface module is configured to test a functionality of the at least one medical device. Thus, the interface module can comprise a control unit to check the signals from the medical devices and to control, whether the sensors work correctly and are correctly attached to the patient. In this embodiment, a connection of the patient table to the trolley can be omitted.

In an embodiment of the invention, the table top is a modular table top and configured to adapt the patient bed to an individual positioning of the patient. A modular table top can be prepared for different scans and applications. The table top can be configured with a mattress for individual positioning of the patient with respect to the envisaged imaging procedure. For example, mammography might require a different positioning than orthopaedic imaging, or therapy like MRLINAC. The table top can also be configured to support prone or supine positioning. For autonomous and parallel preparation of a patient, different combinations of receive coil setups may need to be selected as a function of the region to be imaged or an individually selected anatomy.

In an embodiment of the invention, the patient bed is configured to be used in a computed tomography apparatus, a magnetic resonance imaging system, positron emission tomography, X-ray imaging, ultrasound, and/or therapy systems like MR LINAC, MR hyperthermia, and combinations thereof. The patient bed can also be compatible with different field strength in MRI like, for example, 1.5T or 3T. Compatibility can be realized by introducing an optical fiber link to make the system EMC compatible. Integration of a wireless data link in the patient bed can directly provide near field non galvanic data communication. The modular table top concept can be compatible for MRI as well as CT. If a patient needs a MRI scan directly after a CT scan, the modular concept allows to transport the patient in the preparation unit for multiple patient imaging and to reconfigure the table top. The patient bed can also be configured to be compatible with therapy systems like MR link or a hybrid MRI like MR-PET for therapy or an MR combination with other imaging or triggering techniques like ultrasound. In addition, compatibility with a mobile MRI or diagnostic scanner, which travels to the patient bed can be provided. The patient bed of the invention can also be applied to X-Ray systems. Medical devices can be smart radiation shields, dose monitoring, or portable detectors, for example.

In an embodiment of the invention, the patient bed comprises a power connector configured to receive electric power from the trolley and/or from the medical imaging system. Thus, the patient bed can allow the check and test of the device functionality without any further connection to an external control unit.

In an embodiment of the invention, the power connector is an inductively coupled power transmission. This connection is able to transfer electric power without any wired connection to the patient bed.

According to another aspect of the invention, there is provided a method of imaging a patient in a medical imaging system with a patient bed according to any of the preceding embodiments. The method comprises the steps of providing the patient bed, mounting the patient bed on a trolley, and accommodating the patient on the patient bed. The method comprises further the steps of attaching at least one medical device to the patient and connecting the medical device to the first interface of the interface module thereby preparing the patient for the medical imaging, providing a connection of the second interface of the interface module with a control unit of the trolley, and checking a functionality of the at least one medical device by means of the control unit. Further, the method comprises the steps of transporting the patient bed to the medical imaging system, moving the patient bed from the trolley to the medical imaging system, thereby disconnecting the second interface of the interface module from the trolley and connecting it to the medical imaging system, and acquiring an image of the patient with the medical imaging system. The results of the check of the device functionality can monitored and controlled by a local operator or by a remote operator. The operator can be supported by an artificial intelligence unit.

In an embodiment of the invention, a plurality of patients is prepared for the medical imaging in parallel on a plurality of patient beds. Thus, the patient transport can be equipped for multiple patient preparation. This can increase the throughput of patients through the imaging system.

In an embodiment of the invention, a software algorithm guides a clinician through the steps of the method. For the workflow process of multiple patient preparation and imaging the management process needs to be solved. Thus, it has to be predicted and decided, which parallel and individual processes of preparation, transportation, and docking of the modular patient table have to take place. There are many processes to be controlled in parallel and serial processes such as patient bed configuration and individual patient preparation. The software algorithm can support the clinician and instruct which step and actions have to be taken.

Thus, the benefits provided by any of the above aspects equally apply to all of the other aspects and vice versa.

In a gist, the invention relates to a patient bed for accommodating a patient in a medical imaging system. The patient bed comprises a table top configured for receiving the patient and an interface module comprising a first interface and a second interface. The first interface is configured to be communicationally connected to at least one medical device attached to the patient, and the second interface is configured to be communicationally connected to a control unit for communicating with the at least one medical device. Thus, it is possible to prepare multiple patients in parallel on multiple patient beds before transporting the patients to the medical imaging apparatus. Sensors and coils can be attached to the patient and there functionality can be tested in advance before e4ntering the scanner room for medical imaging.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic setup of a patient bed according to an embodiment of the invention.
Fig. 2 shows a schematic setup of a patient bed with a trolley and a medical imaging system according to an embodiment of the invention.
Fig. 3 shows a scheme of parallel preparation and time scheduling of multiple patients.
Fig. 4A shows a host state machine for controlling the parallel workflow preparation and scanning.
Fig. 4B shows an algorithm of a feedback-reaction loop-model for parallel preparation and imaging workflow.
Fig. 5 shows parallel preparation of multiple patients with medical devices.
Fig. 6 shows a transportation and allocation process of individual patients to individual medical scanners.
Fig. 7 shows a block diagram of a method of imaging a patient in a medical imaging system with a patient bed according to an embodiment of the invention.

### DETAILED DESCRIPTION OF EMBODIMENTS

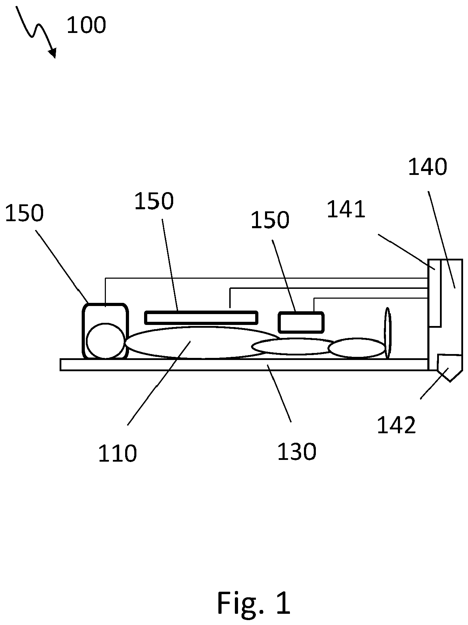

Fig. 1 shows a schematic setup of a patient bed 100 according to an embodiment of the invention. A patient 110 is accommodated on a table top 130 of the patient bed 100. Several medical devices 150 like sensors for detecting physiological signals of the patient 110 or coils of a medical imaging system 120 are applied to the patient 110. The patient bed 100 comprises an interface module 140 with a first interface 141. The medical devices 150 are connected to the first interface 141. The interface module 140 comprises a second interface 142. With this second interface 142, the patient bed can be communicationally connected to a control unit 160 configured to control and readout the medical devices 150.

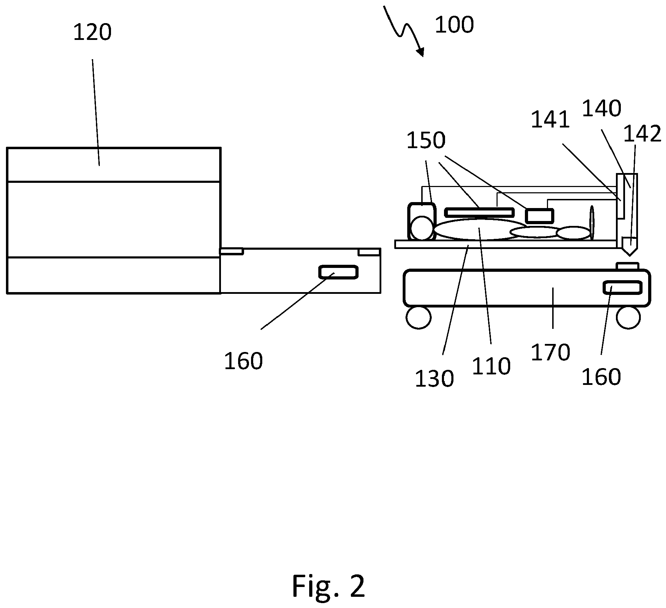

Fig. 2 shows a schematic setup of a patient bed 100 with a trolley 170 and a medical imaging system 120 according to an embodiment of the invention. In addition to the patient bed 100 with the patient 110 and the medical devices 150 attached to the patient, Fig. 2 shows a trolley 170 with the patient bed 100 arranged on the trolley 170. The trolley 170 comprises a control unit 160. When the patient bed 100 is located on the trolley, a connection from the second interface 42 of the interface module 140 to the control unit 160 of the trolley 170 can be established. Thus, in combination with the connection via the first interface 141, the control unit 160 can test and check the signals of the medical devices 150 like sensors and coils. If the patient 110 with the table top 130 of the patient bed 100 is transferred from the trolley 170 to the medical imaging system 120, the communicational connection of the second interface 142 with the control unit 160 of the trolley 170 is disconnected, and a connection of the second interface 142 with a control unit 160 of the medical imaging system 120 is established. Thus, the medical imaging system 120 can readout the sensors and control the coils of the medical devices 150 during the imaging procedure. With this patient bed 100 according to this embodiment of the invention, parallel preparation of multiple patients 110 for medical imaging can be realized.

Docking to the scanner can be achieved via different connection devices. Coils and sensing devices can be directly connected to the patient bed. The patient bed can be connected to the trolley for power supply and digital data transfer. The patient bed can be connected to a diagnostic scanner via an automatic connection process. The sensing devices on the modular patient table need to be connected and linked to the scanner. Local navigation support to precisely fit the bed with the scanner-interface is required. Data connection and power supply connection to the scanner without interaction of staff can be realized. Docking to the scanner can be realized using a connecting device for data and power supply management. The operator does not need to connect manually a connector between the magnet and the trolley. An interface can be used, which is partly galvanic, optical or wireless. When the patient bed is shifted from the trolley to the magnet docking device of the imaging system, the patient bed including the sensors is controlled by the imaging system. Data flow from the sensors and power supply is electrically switched from the smart trolley to the magnet. A special connection to a transport unit as well as docking connection to the diagnostic scanner unit can be achieved. For example, a galvanic connection transmitting power and data, an optical fiber or free space optics for data transmission, an inductively coupled power transmission, or radio frequency data transmission are possible. The following options as gadgets to be applied for parallel preparation of multiple patients in the preparation room can be provided: A contrast injector for CT or MR, a needle to the patient, a pump integrated in patient bed, a sedation unit, wireless sensors, an ultrasound system, or orthopaedic markers in the preparation-room

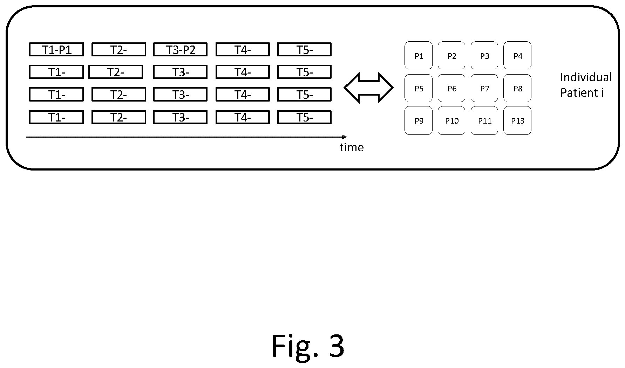

Fig. 3 shows a scheme of parallel preparation and time scheduling of multiple patients 110. This may be achieved using a real time planning and mapping software module.

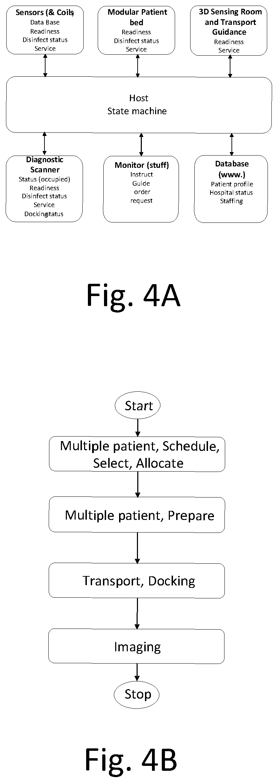

Fig. 4A shows a host state machine for controlling the parallel workflow preparation and scanning. The software algorithm controls the workflow of parallel patient preparation and imaging. Different software modules may be active in parallel and interact, control each other and exchange data. An inventory of AI data learning modules can be provided to predict in advance optimal coil fit, configuration, and timing of transportation. A patient queue for multiple imaging systems like scanners may need to be organized by an overall workflow management process. Preparation rooms to access multiple scanners need to be organized, arranged, and predicted. Availability and readiness of systems to be used for preparation need to be checked in real time. Transparency of status of RF coils is inherently important regarding disinfection status and readiness, like whether the coil is charged, the electrical status protocol, or the service protocol. Such a system may need a special algorithm, which may be based on an artificial intelligence to organize, control, predict, and decide, and with input data from sensors, or from a data base.

Fig. 4B shows an algorithm of a feedback-reaction loop-model for parallel preparation and imaging workflow. Multiple patients 110 to be imaged are scheduled, their imaging process is selected and they are allocated to a preparation room and a medical scanner. The patients 110 are prepared, they are positioned on a respective patient bed 100 and the required medical devices are attached to the patients 110 and connected to the interface module 140 of the patient bed 100. After that, the patients 110 are transported and docked to the medical imaging system 120. After imaging, the process is stopped.

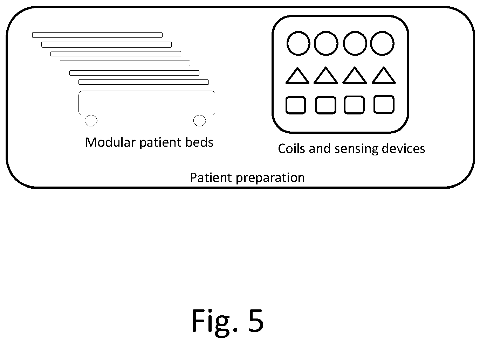

Fig. 5 shows parallel preparation of multiple patients 110 with medical devices 150. The modular patient beds 100 need to be configured for the upcoming or predicted scan. The configuration may depend on the clinical protocol, like mammography, cardiac or orthopaedic scans, the position of the patient 110, and the predicted number of scans in a defined timeslot. The software management may decide about the number and individual equipment needed. A staff member can get the information on how, when and which medical devices 150 such as coils or sensors have to be applied to configure the patient bed 100 during a defined timeslot. A display device on the patient bed 100 or the trolley 170 can monitor the state and information to the operator or staff.

During the preparation of each patient 110 the individual patient profile can be considered. The preparation in the preparation room has the function of setting and locating devices and gadgets such as RF coils, sensors for ECG, PPU and respiration, contrast injectors, sedation injectors, oxygen supply of the patient, or blood vessel access for sedation and stabilization, or therapy devices such as local applicators for hyperthermia, a black box monitoring device to monitor imaging parameters like electromagnetic fields, or dosimeters, based on a defined protocol.

The individual devices are connected and locally checked and feedback of functionality and sensor data flow is transmitted to the workflow management system to configure upcoming diagnostic imaging protocols on the imaging scanners. During preparation it might not be exactly clear, which scanner is allocated. For allocation a timetable, preferably in real time, decides about allocation. An interrupt process may be necessary, for example if the scanner fails, an emergency at the patient occurs, no medical assistant is available, a software crash occurs, data is not yet available, or idle waiting.

As shown in Fig. 5, a number of multiple modular patient beds is available and need to be controlled for parallel patient preparation. Each bed will be individually equipped with coils and sensing devices. All coils can be managed by a service and disinfection workflow system, which coil corresponds to which scanner and clinical scan. For example, garment coils need special disinfection and cleaning process. Different coils may be dressed correctly in the preparation room including monitoring and a function test. If coil-garment is not working properly, the coil-garment may need to be changed. If there are many coils in the preparation room, the patients or the operator can select individually sized coils. The coil can even belong to different vendor systems, which might be not compatible.

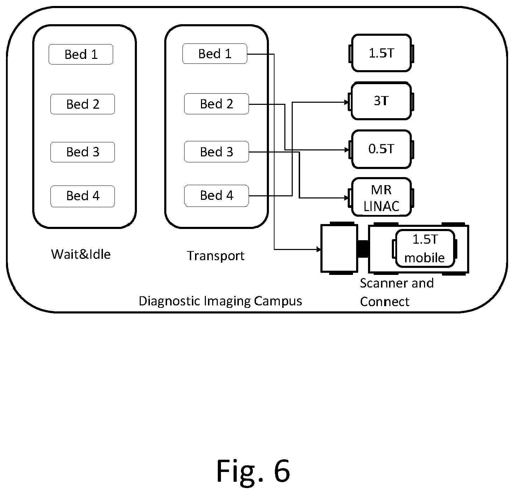

Fig. 6 shows a transportation and allocation process of individual patients 110 to individual medical imaging systems 120. When a scanner of a pool of scanners is about to be ready, then the process of transportation can be activated for the selected patient. The transportation process may be fully automated or guided with a medical assistant. Clear identification process of the selected patient and scanner bed needs to be solved. The table top 130 with the patient 110 and active and passive sensing units may need to be electrically connected for data control exchange and power supply. The transport may be interrupted in case of multiple patient workflow problems like scanner problems, emergency of the patient, no staff, etc. As shown in Fig. 6, the patient bed 100 with a prepared patient 110 is ready to scan and final allocation is realized, when a scanner is ready to use. The scanner can have different status like occupied, scanning, docking, undocking, disinfecting, ready, service, etc. A patient bed can have status like free, occupied, under preparation, device checking, transport to scanner, disinfected, or service.

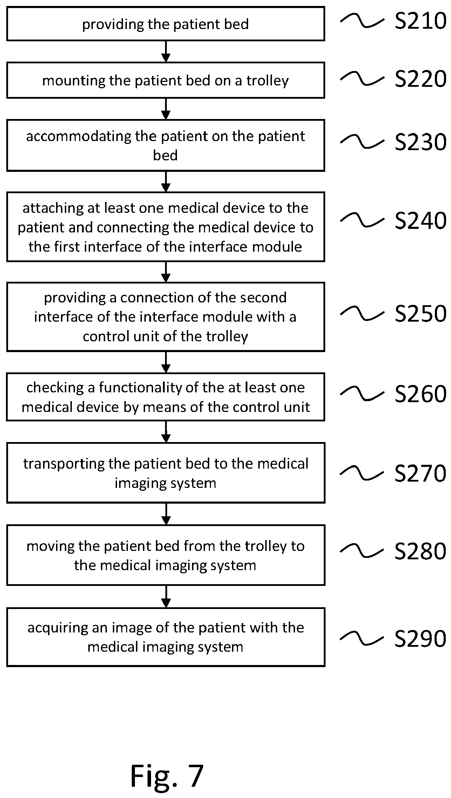

Fig. 7 shows a block diagram of a method of imaging a patient 110 in a medical imaging system 120 with a patient bed 100 according to an embodiment of the invention. The method comprises the step S210 of providing the patient bed 100, the step S220 of mounting the patient bed 100 on a trolley 170, and the step S230 of accommodating the patient 110 on the patient bed 100. After step S240 of attaching at least one medical device 150 to the patient 110 and connecting the medical device 150 to the first interface 141 of the interface module 140 thereby preparing the patient 110 for the medical imaging, step S250 of providing a connection of the second interface 142 of the interface module 140 with a control unit 160 of the trolley 170 is performed. However, step S240 can also be performed after step S250. Further, the method comprises the step S260 of checking a functionality of the at least one medical device 150 by means of the control unit 160, and step S270 of transporting the patient bed 100 to the medical imaging system 120. In addition, step S280 of moving the patient bed 100 from the trolley 170 to the medical imaging system 120, thereby disconnecting the second interface 142 of the interface module 140 from the trolley 170 and connecting it to the medical imaging system 120, and step S290 of acquiring an image of the patient 110 with the medical imaging system 120 are performed.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: patient bed
- 110: patient
- 120: medical imaging system
- 130: table top
- 140: interface module
- 141: first interface
- 142: second interface
- 150: medical device
- 160: control unit
- 170: trolley

## Claims

1. A patient bed (100) for accommodating a patient (110) in a medical imaging system (120), the patient bed comprising:
a table top (130) configured for receiving the patient (110); and
an interface module (140) comprising a first interface (141) and a second interface (142), wherein the first interface (141) is configured to be communicationally connected to at least one medical device (150) attached to the patient (110), and wherein the second interface (142) is configured to be communicationally connected to a control unit (160) for communicating with the at least one medical device (150).

2. The patient bed (100) according to claim 1, wherein the control unit (160) is part of the medical imaging system (120), or wherein the control unit (160) is part of a trolley (170) for transporting the patient bed (100).

3. The patient bed (100) according to claim 2, wherein the second interface (142) is configured to disconnect from the trolley (170) and to connect with the medical imaging system (120) when the patient bed (100) is moved from the trolley (170) to the medical imaging system (120).

4. The patient bed (100) according to any of the preceding claims, wherein the at least one medical device (150) is a physiological sensor for detecting a body function of the patient (110), a coil of the medical imaging system (120), or a device for treating the patient (110).

5. The patient bed (100) according to any of the preceding claims, wherein the second interface (142) is configured to establish the connection to the control unit (160) automatically.

6. The patient bed (100) according to claim 5, wherein the connection to the control unit (160) is established using 3D sensing.

7. The patient bed (100) according to any of the preceding claims, wherein the connection of the first interface (141) with the at least one medical device (150) and/or the connection of the second interface (142) with the control unit (160) is a galvanic, an optical, or a wireless connection.

8. The patient bed (100) according to any of the preceding claims, wherein the interface module (140) is configured to test a functionality of the at least one medical device (150).

9. The patient bed (100) according to any of the preceding claims, wherein the table top (130) is a modular table top and configured to adapt the patient bed (100) to an individual positioning of the patient (110).

10. The patient bed (100) according to any of the preceding claims, wherein the patient bed (100) is configured to be used in a computed tomography apparatus, a magnetic resonance imaging system, positron emission tomography, X-ray imaging, ultrasound, and/or therapy systems like MR LINAC, MR hyperthermia, and combinations thereof.

11. The patient bed (100) according to any of the preceding claims, wherein the patient bed (100) comprises a power connector configured to receive electric power from the trolley (170) and/or from the medical imaging system (120).

12. The patient bed (100) according to claim 11, wherein the power connector is an inductively coupled power transmission.

13. Method of imaging a patient (110) in a medical imaging system (120) with a patient bed (100) according to any of claims 1 to 12, the method comprising the steps of:
providing the patient bed (100);
mounting the patient bed (100) on a trolley (170);
accommodating the patient (110) on the patient bed (100);
attaching at least one medical device (150) to the patient (110) and connecting the medical device (150) to the first interface (141) of the interface module (140) thereby preparing the patient (110) for the medical imaging;
providing a connection of the second interface (142) of the interface module (140) with a control unit (160) of the trolley (170);
checking a functionality of the at least one medical device (150) by means of the control unit (160);
transporting the patient bed (100) to the medical imaging system (120);
moving the patient bed (100) from the trolley (170) to the medical imaging system (120), thereby disconnecting the second interface (142) of the interface module (140) from the trolley (170) and connecting it to the medical imaging system (120); and
acquiring an image of the patient (110) with the medical imaging system (120).

14. The method according to claim 13, wherein a plurality of patients (110) is prepared for the medical imaging in parallel on a plurality of patient beds (100).

15. The method according to any of claims 13 or 14, wherein a software algorithm guides a clinician through the steps of the method.
